Europäisches Patentamt

European Patent Office

Office européen des brevets

(19)

(11) Publication number: **0 169 001**
A2

(12) # EUROPEAN PATENT APPLICATION

(21) Application number: **85304670.4**

(51) Int. Cl.⁴: **A 61 L 27/00, A 61 F 2/30**

(22) Date of filing: **01.07.85**

(30) Priority: **17.07.84 US 631570**

(43) Date of publication of application: **22.01.86
Bulletin 86/4**

(84) Designated Contracting States: **AT BE CH DE FR GB IT
LI LU NL SE**

(71) Applicant: **COLLAGEN CORPORATION, 2500 Faber
Place, Palo Alto, California 94303 (US)**

(72) Inventor: **Sabelman, Eric, 711 Central Avenue, Menlo
Park California 94025 (US)**
Inventor: **Piez, Karl, 1120 Bay Laurel Drive, Menlo Park
California 94025 (US)**

(74) Representative: **Harrison, David Christopher et al,
MEWBURN ELLIS & CO 2/3 Cursitor Street, London
EC4A 1BQ (GB)**

(54) **Collagen coated bone implants.**

(57) Improved prostheses which are secured to surrounding
bone by growth of surrounding bone tissue are described. The
ingrowth of surrounding bone is encouraged by coating the
stress-bearing member of the prostheses with a purified,
sterile, non-immunogenic collagen preparation.

-1-

## COLLAGEN COATED BONE IMPLANTS

The invention herein relates to the field of correcting bone defects by implanting stress-bearing bone replacements. In particular, the invention relates to the use in such procedures of collagen coated prostheses.

The use of stress-bearing materials for bone replacement is, of course, by this time, well known. A large number of designs have been used to replace missing or diseased portions of the bone structure which are stress-bearing such as bone shafts, joints, and tooth roots. These designs include artificial shafts and joints and associated devices intended to mimic the functionality of the human skeletal system. The designs can be quite elaborate. See, for example, U.S. 3,820,167 which discloses a design for an artificial hip joint. Alternatively, attempts have been made to substitute preserved bone as a transplant from other species. See, for example, U.S. 3,126,884 which discloses method of preserving a bone transplant in plasma and antibiotics, and U.S. 3,318,774 which discloses a method of preparing such a transplant through a series of steps to extract fats and sterilize the material.

More commonly, however, shafts or other prostheses constructed of metal or metal alloys have been used as the stress-bearing portion of the bone replacement. Such metal pins or artificial joints are constructed of suitable inert metals such as titanium, stainless steels, other alloys such as cobalt, chromium and molybdenum alloys. The metallic pin or joint is

-2-

sometimes provided with an oxide coating in order to prevent corrossion and instability.

It has been observed in the use of such metallic implants, that it is frequently necessary to provide a mechanism to prevent the shifting of the implant in place and to fix its position with respect to adjacent bone. Attempts have been made to provide this fixation using a cement; indeed one form of such cement involving collagen as one component is disclosed in USSR patent application 733665 published 15 May 1980 wherein a mixture of cartilage, collagen of unknown origin, and the patient's own blood is used to secure the implanted pin in place.

Coating the prosthesis with a material which purportedly encourages bone growth, such as a "bioglass" thus securing the implant with the ingrowth, has been disclosed (French Appln Publn No 2,383,656 published 13 October 1978). It has also been attempted to use wire mesh surrounding the stress-bearing member of the prosthesis to provide sufficient flexibility to obtain a firm fit, in some cases aided by additional metal strips. See EPO Publication 0071242, published 9 February, 1983. In connection with this mesh, a biodegradeable, biocompatable material, one example of which is a collagen, again of unknown origin is employed, apparently to prevent damage to the wire mesh during insertion of the implant. The resulting implant eventually is secured by additional bone growth. Indeed, research has consistently shown that prostheses which have porous metal or porous ceramic surfaces are better secured by bony growth into this porous surface.

An attempt has been made to simulate this bony ingrowth by providing a bone particle coating. U.S. patent 3,918,100 describes prostheses made of various

-3-

metals which are coated using RF sputtering with bone particles in a vaccuum. In this disclosure, aluminum oxide substrates were coated with a powder made of lye-treated bone chips. The coated prostheses are said to encourage living bone to grow onto the implant, and the coating finally to be absorbed by the surrounding tissue.

None of the foregoing represents a satisfactory solution. The materials used are often immunogenic and not capable of conducting or stimulating surrounding bone growth at a sufficient rate to prevent damage from shifting of the prostheses. The present invention provides prostheses which simulate the intrusion of bone at sufficient rates to obtain a more satisfactory result.

It is clear from present experience that an implanted stress-bearing prosthesis should be provided with a means to assure permanent attachment of the prosthesis to the remaining portions of the skeletal system; ideally, it should be provided with a fixative which encourages the surrounding bone to intrude into the porous surface of the implant. The present invention provides an effective and satisfactory form of fixative.

Accordingly, in one aspect, the invention relates to a prosthesis for implantation as a bone replacement which comprises a stress-bearing member coated with sterile non-immunogenic collagen, advantageously an atelopeptide form of xenogeneic origin. Autogeneic collagen with similar properties is also operable, but is prohibitively scarce to provide the amounts required. To the extent that the collagen is an atelopeptide form it is sufficiently non-immunogenic to permit non-autogeneic forms to be used. Accordingly, the xenogeneic aspect of the collagen provided in the

-4-

invention is intended to indicate that the majority of the material can be of foreign species origin and includes such coated materials which may contain some percentage of autogenous material.

In other aspects, the invention relates to a method of repairing bone defects which employs the prosthesis of the invention and to methods of preparing the prosthesis.

Modes of Carrying Out the Invention

A. Definitions

"Free from impurities" or "purified" refers to those impurities which are normally associated with the collagen or other preparation in its natural state. Thus, collagen prepared from calf skin is free from impurities when other components with calf skin have been substantially removed; that from bone, when other components are eliminated.

"Reconstituted" collagen refers to collagen which has been disassembled into individual triple helical molecules with or without their telopeptide extensions and brought into solution, and then regrouped into "fibrillar" form. In this form, the fibrills consist of long thin collagen molecules staggered relative to one another by multiples of about ¼ their length. This results in a banded structure which can be further aggregated into fibers.

"Zyderm® collagen implant" (ZCI) refers to a purified atelopeptide preparation of collagen derived from calf skin. It is a reconstituted fibrillar collagen attainable from Collagen Corporation, Palo Alto, California.

"Bone collagen powder" (BCP) is a purified atelopeptide preparation of collagen derived from demineralized bone.

"Collagenfleece®" refers to a purified, non-reconstituted form of collagen derived from pig skin as described in U.S. 4,066,083. It apparently still contains telopeptides and is not as useful as the atelopeptide forms. It is prepared in freeze-dried form and sterilized by $\gamma$-irradiation.

B. Detailed Description

B.1. The Stress-Bearing Member of the Prosthesis

The prostheses of the invention comprise a stress-bearing member coated with a form of collagen with the required properties of sterility and non-immunogenicity. The stress-bearing member per se does not constitute the invention, and any conventionally used or operable stress-bearing prosthesis can be used. Such prostheses are ordinarily metallic or ceramic and may be provided with porous surfaces by suitable techniques known in the art. The stress-bearing members themselves range from simple pins used to replace bone shaft portions to fairly complex artificial joints and artificial tooth roots. These designs may be made of ceramic or metal, or, indeed, transplanted replacement bone. They are of such design and material as to be appropriate to their intended use which may include replacement of diseased bone, correction of defects, or anchoring teeth.

B.2. Collagen Preparation

Native collagen consists in large part of a triple helical structure containing repeating triplet sequences composed of glycine linked to two additional amino acids, commonly proline and hydroxyproline; thus, glycine appears in every third position in the chain. In addition, all collagen chains contain regions at each end which do not have the triplet glycine sequence and are thus not helical. These regions are thought to be responsible for the immunogenicity associated with most collagen preparations. Immunogenicity can, in large part, be mitigated by removal of these regions to produce "atelopeptide" collagen. This can be accomplished by digestion with proteolytic enzymes such as trypsin or pepsin. The non-helical telopeptide regions are also required to form the cross links which are responsible for stability of the fibrillar structure as they contain aldehydes capable of crosslinkage; atelopeptide collagen must be crosslinked artificially if it is desired to obtain this characteristic.

As the collagen coating which forms part of the prosthesis of the invention can be atelopeptide collagen, it will have the desired properties of being non-inflamatory and non-immunogenic. As all atelopeptide collagen has these properties, a xenogeneic form of collagen can be used. While the use of autogenous collagen is not precluded, permissive xenogeneity permits an economic application of the invention. It is clearly much more desirable to be able to use collagen from an animal, such as bovine or porcine sources than to require the use of this material derived from human beings. In extreme cases, failure to provide a collagen preparation with these properties in any degree may result in the requirement that collagen derived from the same individual as is being treated be used. This clearly becomes counterproductive and impractical.

-7-

The requirements for the collagen preparation used to coat the stress-bearing member are merely that the collagen be a non-immunogenic, preferably atelopeptide collagen, which is uncontaminated with non-collagenous materials -- i.e., purified, and which is sterile so as not to produce infection. Sterilization can be accomplished by a variety of means including, for example, heat, irradiation, or, if the preparation is reconstituted, by sterile filtration of the collagen while it is still in solution. Sterilization by heat or $\gamma$-irradiation may cause degradation or crosslinking; however it is not clear that these results will have a negative impact on the efficacy of the resulting preparation. Accordingly, any method of sterilization suitable for the preparation used is satisfactory.

A variety of sterile purified non-immunogenic (atelopeptide) collagen preparations are known in the art. Especially preferred is the preparation known as Zyderm® collagen implant (ZCI), a reconstituted preparation made from calf skin, and commercially available. It is provided in the form of a gel.

Another form of collagen which can be utilized is a bone collagen powder (BCP) which is derived from demineralized bone, by procedures similar to those of Oliver and Grant, described in British patent application GB 1565340A. Briefly in general, bone, for example, bovine, porcine, or other mammalian bone, preferably compact bone, is cleaned, frozen, pulverized and demineralized in hydrochloric acid, or other suitable acid, using standard techniques. The residual organic matter is separated and digested using proteolytic enzymes sequentially or in combination. Neutral proteases, e.g., trypsin, which allow for the selective degradation of non-collagenous proteins are preferred;

certain acid proteases such as pepsin cause partial digestion of the collagen. Non-proteinaceous bone components are also removed using suitable enzymes such as chondroitinase, hyaluronidase, and various nucleases. After treatment with the appropriate enzymes, the insoluble material consists of purified atelopeptide collagen in the form of BCP. It may be used after sterilization by known methods such as γ-irradiation or heat. A further description of BCP is set forth in copending application Collagen docket number COL0039P/055 and incorporated herein by reference. As this preparation still contains the telopeptides, it is less desirable than those described above.

Also commercially available is a form of collagen drived from pig skin, Collagenfleece*. This is generally supplied in the form of a freeze dried solid, but can be resuspended for coating prostheses if desired.

Of course, it is not necessary that all of the collagen used to coat the prosthesis be of one type of preparation. Accordingly, mixtures in any proportion of the foregoing purified collagen preparations or other collagen preparations which meet the required specifications of being non-immunogenic, pure, and sterile can be used. The proportions are not critical and can be adjusted to suit he desired physical properties of the coating. Indeed, an additional mode of preparation, for example, different from any of the foregoing, may be used to obtain purified collagen, which can then be treated by appropriate proteases to remove the atelopeptides. This mode of preparation is disclosed in EPO application 0012959, published 9 July 1980.

-9-

### B.3. Process for Coating the Prosthesis

A number of procedures may be used to coat the prosthesis, but the preferred and simplest procedure is to paint on or dip the prosthesis in a suspension of the collagen protein containing about 5-100 mg/ml, preferably about 30-70 mg/ml, at the time of surgery. Sufficient collagen is applied to fill the gaps between implant and host bone.

Alternatively, a suspension of the collagen can be air-dried or freeze-dried in the form of a sheet which is wrapped around the implant and inserted with it at the time of surgery.

It is not ruled out that the above procedures could be done prior to surgery and the coated implant properly packaged to retain the collagen coating.

### B.4. Implantation

Implantation of the prosthesis employs techniques appropriate for the particular defect to be remedied. These procedures are those ordinarily used in the art and do not form part of the invention.

The efficacy of the coating in encouraging suitable bone growth into the surface of the prosthesis may be either conductive or inductive depending on the nature of the collagen preparation used and whether or not it contains additional proteins such as those normally characterised as osteogenesis factors.

Conductive bone growth refers to a process for encouraging bone growth which involves the metabolism of previously committed osteoprogenitor cells, and the osteogenesis is directly effected by these cells. Ordinarily this can occur by providing a matrix into which the new bone can conveniently grow. If this is the mechanism whereby the coating encourages growth, it

-10-

simply provides such a matrix. Inductive bone growth further includes the step of converting previously uncommitted cells to osteogenic ones. The precise nature of this process is not known, but it is understood that it is mediated by proteins found ordinarily in bone. Purification of such factors is disclosed in U.S. patents 4,294,753 and 4,434,094. If desired, the collagen coating of the prosthesis may include such factors and in this case bone growth will be considered inductive, though induction includes the conductive process supported by the matrix provided by the collagen.

C. Examples

The following examples are intended to illustrate but not to limit the invention.

C.1. Preparation of the Collagen Coating Suspension

Zyderm® collagen implant was used as the reconstituted fibrillar atelopeptide component. The reconstituted native type collagen fibrils were used at a concentration of about 55 mg/ml. The preparation had been sterilized during processing by filtration and thus was not subjected to degradative procedures such as heat or irradiation.

C.2. Preparation of the Coated Prosthesis

A stainless steel shaft, shaped to replace a portion of femur is repeatedly dipped into the suspension of the ZCI prepared in ¶C.1, and allowed to dry. This results in a thin coating over the entire surface of a dried collagen network.

-11-

C.3.  Implantation of the Prosthesis
      of the Invention

The diseased or damaged area of the femur to receive the implant is cleared of marrow and debris, and the coated implant fitted into the defect.  Surrounding bone invades the coating, holding the implant in place.

-12-

## Claims

1.  A prosthesis for implantation as a bone replacement which comprises an artificial, non-biologically derived stress-bearing member coated with a purified, sterile, non-immunogenic collagen preparation.

2.  The prosthesis of claim 1 wherein the collagen preparation is an atelopeptide collagen.

3.  The prosthesis of claim 2 wherein the collagen preparation is xenogeneic.

4.  The prosthesis of claim 1 wherein the collagen preparation further includes an osteogenesis factor.

5.  The prosthesis of claim 1 wherein the collagen preparation is reconstituted and fibrillar.

6.  The prosthesis of claim 2 wherein the collagen preparation contains ZCI.

7.  The prosthesis of claim 2 wherein the collagen preparation contains BCP.

8.  A method of replacing missing bone which comprises providing an implant comprising the prosthesis of claim 1.

9.  A method of making a prosthesis for bone repair, which method comprises coating an artificial,

-13-

non-biologically derived stress-bearing member with purified, sterile, non-immunogenic collagen.